(19) Europäisches Patentamt
European Patent Office
Office européen des brevets

(11) EP 3 757 085 B1

(12) EUROPEAN PATENT SPECIFICATION

(45) Date of publication and mention of the grant of the patent:
**10.11.2021 Bulletin 2021/45**

(21) Application number: **19305862.5**

(22) Date of filing: **27.06.2019**

(51) Int Cl.:
***C07C 29/143*** *(2006.01)* ***C07C 31/42*** *(2006.01)*
***C07C 45/72*** *(2006.01)* ***C07C 49/173*** *(2006.01)*
***C07B 57/00*** *(2006.01)*

(54) **PERFLUORINATED POLYOLS AND THEIR USE FOR THE ENANTIOMERIC SEPARATION OF CHIRAL ANIONS**

PERFLUORIERTE POLYOLE UND DEREN VERWENDUNG ZUR ENANTIOMEREN TRENNUNG CHIRALER ANIONEN

POLYOLS PERFLUORÉS ET LEUR UTILISATION POUR LA SÉPARATION ÉNANTIOMÉRIQUE D'ANIONS CHIRAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.12.2020 Bulletin 2020/53**

(73) Proprietors:
- **Centre National de la Recherche Scientifique 75016 Paris (FR)**
- **Université d'Aix Marseille 13007 Marseille 7 (FR)**

(72) Inventors:
- **QUINTARD, Adrien 13013 MARSEILLE (FR)**
- **RODRIGUEZ, Jean-Antoine 13016 MARSEILLE (FR)**
- **SPERANDIO, Céline 13002 MARSEILLE (FR)**

(74) Representative: **Cabinet Becker et Associés 25, rue Louis le Grand 75002 Paris (FR)**

(56) References cited:
- **WILLIAM H. PIRKLE ET AL: "Nuclear magnetic resonance determination of enantiomeric composition and absolute configuration of .gamma.-lactones using chiral 2,2,2-trifluoro-1-(9-anthryl)ethanol", JOURNAL OF ORGANIC CHEMISTRY, vol. 42, no. 2, 1 January 1977 (1977-01-01), pages 384-387, XP055648673, US ISSN: 0022-3263, DOI: 10.1021/jo00422a061**
- **MASOUD SAMET ET AL: "Power of a Remote Hydrogen Bond Donor: Anion Recognition and Structural Consequences Revealed by IR Spectroscopy", JOURNAL OF ORGANIC CHEMISTRY, vol. 80, no. 2, 29 December 2014 (2014-12-29), pages 1130-1135, XP055648675, US ISSN: 0022-3263, DOI: 10.1021/jo502652z**

EP 3 757 085 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The invention relates to perfluorinated polyols and their use for enantiomeric separation of chiral anions. It also relates to a process for preparing such perfluorinated polyols, and to a process for the enantiomeric separation of chiral anions.

### TECHNICAL BACKGROUND

Anion coordination is central to a broad range of applications, including sensing, anion extraction, catalysis and smart materials. It also plays a prominent role in the development of innovative strategies, due to the crucial regulation role of anions, such as chloride anions, in the body.

The anion coordination properties of a compound are known to be closely related to its structure, and more particularly, to its spatial conformation. In this regard, several structural families of coordinating compounds have been developed over the last few years.

Pirkle et al. (J. Org. Chem. 1977, 42, 384-387) discloses the use of chiral 2,2,2-trifluoro-1-(9-anthryl)ethanol for the asymmetric resolution of chiral lactones.

Samet et al. (J. Org. Chem. 2015, 80, 1130-1135) describe trifluoromethylated cyclic polyols having a *scyllo*-inositol mono-orthoformate structure. It is demonstrated that the rigidity of the structure imposed by the cyclic nature of the molecule combined with the hydrogen bonding ability of the alcohol groups allows these compounds to efficiently bind anions. In particular, a chloridebinding constant of $10^6$ $M^{-1}$ in acetonitrile is obtained with the compound represented by the following formula (1):

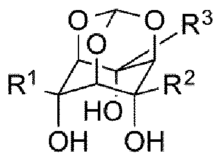 (1),

wherein $R^1$, $R^2$, and $R^3$ are $CF_3$ groups.

Shokri et al. (J. Am. Chem. Soc. 2013, 135, 9525-9530) describe the physico-chemical properties, and more particularly the Brønsted acidity, anion binding and catalytic abilities of the following trifluoromethylated acyclic polyols (2), (3) and (4):

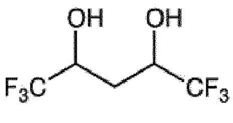 (2), 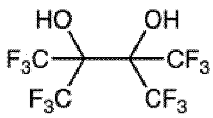 (3), 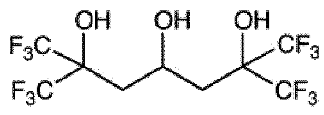 (4).

Despite their acyclic structure, it is demonstrated that diols (2) and (3) can bind chloride anions, with high binding constants of 3300 $M^{-1}$ and 6700 $M^{-1}$ respectively. Triol (4) appears as a good Bronsted acid catalyst, especially in a Friedel-Crafts reaction between β-nitrostyrene and N-methylindole, however no anion-binding constant is provided for this compound.

Compounds (1), (2) and (3) described above have proven to be efficient anion-binding agents, however such compounds suffer from similar disadvantages. In particular, such compounds are achiral and/or are prepared according to difficult and non-stereoselective processes, which limit their use in many applications and above all, prevents their use in the enantiomeric separation of chiral anions.

Thus, there remains a need to provide efficient chiral anion-binding agents which can easily be prepared in a stereoselective fashion.

### SUMMARY OF THE INVENTION

In this respect, the inventors have prepared innovative chiral triols having two perfluorinated aliphatic side chains, according to a highly stereoselective, efficient and simple process. It has been demonstrated that such compounds have a high ability to coordinate anions. More specifically, tests with a reference anion, namely the chloride anion, have

shown that anion-binding constants of up to 4400 $M^{-1}$ could be reached. The use of one specific stereoisomer has allowed to extend this ability to the enantiomeric separation of chiral anions, through a selective enantiomeric coordination to a preferential enantiomer.

Thus, the invention relates to a compound represented by the following formula (I):

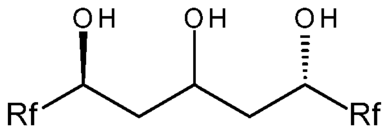

(I),

wherein Rf represents a $C_1$-$C_{12}$ perfluorinated aliphatic chain,
and/or its enantiomer.

It also relates to a use of a compound of formula (I), for the enantiomeric separation of chiral anions.

It further relates to a process for the enantiomeric separation of chiral anions, comprising the steps of:

(a) contacting a compound of formula (I) with a mixture comprising a pair of enantiomers of a chiral anion, so as to obtain a complex (C) consisting of said compound of formula (I) and one enantiomer of said pair;
(b) isolating said complex (C) obtained in step (a); and
(c) optionally recovering the enantiomer from the complex (C) obtained in step (b).

Another object of the present invention is a process for preparing a compound of formula (I), comprising the steps of:

(a) reacting 1,3-acetonedicarboxylic acid with an aldehyde of formula Rf-CHO, wherein Rf represents a $C_1$-$C_{12}$ perfluorinated aliphatic chain, or a hydrate thereof, in the presence of a copper salt and a chiral bis(oxazoline) ligand; and
(b) reacting the compound obtained in step (a) with a hydride-type reducing agent.

## BRIEF DESCRIPTION OF THE FIGURES

Figure 1 shows the $^1$H NMR chemical shift of hydroxy groups of compound (2) according to the concentration of TBA((S)-BINOL-$PO_4$).
Figure 2 shows the $^1$H NMR chemical shift of hydroxy groups of compound (2) according to the concentration of TBA((R)-BINOL-$PO_4$).

## DETAILED DESCRIPTION OF THE INVENTION

### Definitions

In the following description, the expression "comprised between" is intended to include the upper and lower limits within the range described.

The words mentioned herein with a prefix such as $C_1$-$C_{12}$ can also be used with lower numbers of carbon atoms such as $C_1$-$C_2$, $C_1$-$C_9$, or $C_2$-$C_5$. If, for example, the wording $C_1$-$C_{12}$ is used for an aliphatic chain, then said aliphatic chain may comprise from 1 to 12 carbon atoms, especially 1, 2, 3, 4, 5, 6, 7, 8, 9, 10, 11, or 12 carbon atoms.

"Aliphatic chain" refers to a linear or branched, cyclic or acyclic, non-aromatic hydrocarbon chain, optionally comprising at least one double bond and/or at least one triple bond. A "hydrocarbon aliphatic chain" may in particular be an alkyl, alkenyl, alkynyl, cycloalkyl group.

"Alkyl" refers to a saturated, linear or branched acyclic hydrocarbon group. Examples of $C_1$-$C_{12}$ alkyl include, but are not limited to, methyl, ethyl, propyl, isopropyl, butyl, isobutyl, *tert*-butyl, pentyl, hexyl, heptyl, octyl, nonyl, decyl, undecyl, or dodecyl. Examples of $C_1$-$C_6$ alkyl include, but are not limited to, methyl, ethyl, propyl, isopropyl, butyl, isobutyl, *tert*-butyl, pentyl, or hexyl,

"Alkenyl" refers to an unsaturated, linear or branched hydrocarbon group comprising at least one carbon-carbon double bound. Examples of $C_2$-$C_{12}$ alkenyl include, but are not limited to, ethenyl, propenyl, isopropenyl, butenyl, isobutenyl, pentenyl, hexenyl, heptenyl, octenyl, nonenyl, decenyl, undecenyl or dodecenyl. Examples of $C_2$-$C_6$ alkenyl

include, but are not limited to, ethenyl, propenyl, isopropenyl, butenyl, isobutenyl, pentenyl, or hexenyl.

"Alkynyl" refers to an unsaturated, linear or branched hydrocarbon group comprising at least one carbon-carbon triple bound. Examples of $C_2$-$C_{12}$ alkynyl include, but are not limited to, ethynyl, propargyl, butynyl, pentynyl, hexynyl, heptynyl, octynyl, nonynyl, decynyl, undecynyl or dodecynyl. Examples of $C_2$-$C_6$ alkynyl include, but are not limited to, ethynyl, propargyl, butynyl, pentynyl, or hexynyl.

"Cycloalkyl" corresponds to a saturated or unsaturated mono-, bi- or tri-cyclic alkyl group. It also includes fused, bridged, or spiro-connected cycloalkyl groups. Examples of "$C_3$-$C_{12}$ cycloalkyl" or "$C_3$-$C_6$ cycloalkyl" include for instance cyclopropyl, cyclobutyl, cyclopentyl, and cyclohexyl.

"Aryl" corresponds to a mono- or bi-cyclic aromatic hydrocarbon group comprising between 5 and 14 atoms, such as phenyl, biphenyl, or naphthyl.

"Heterocycloalkyl" corresponds to a saturated or unsaturated cycloalkyl group as defined above further comprising at least one heteroatom or heteroatomic group such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, pyranyl, tetrahydropyranyl, thiomorpholinyl, pyrazolidinyl, piperidyl, piperazinyl, azepanyl, 1,4-dioxanyl, imidazolinyl, pyrrolinyl, pyrrolidinyl, piperidinyl, imidazolidinyl, morpholinyl, 1,4-dithianyl, pyrrolidinyl, quinolizinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydro-2H-pyranyl, tetrahydrofuranyl, and tetrahydrothiophenyl. "Heterocycloalkyl" may also refer to a 5-10 membered bridged heterocyclyl such as 7-oxabicyclo[2,2,1]heptanyl.

"Heteroaryl" as used herein corresponds to an aromatic, mono- or poly-cyclic group comprising between 5 and 14 atoms and comprising one or more heteroatoms, such as nitrogen (N), oxygen (O) or sulphur (S) atom, or heteroatomic groups. Examples of such mono- and poly-cyclic heteroaryl group may be: pyridinyl, thiazolyl, thiophenyl, furanyl, pyrrolyl, imidazolyl, triazolyl, tetrazolyl, benzofuranyl, thianaphthalenyl, indolyl, indolinyl, quinolinyl, isoquinolinyl, benzimidazolyl, triazinyl, thianthrenyl, isobenzofuranyl, phenoxanthinyl, isothiazolyl, isoxazolyl, pyrazinyl, pyridazinyl, indolizinyl, isoindolyl, indazolyl, purinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, benzotriazolyl, benzoxazolyl, benzisoxazolyl, oxindolyl, benzothienyl, benzothiazolyl, s-triazinyl, oxazolyl, or thiofuranyl.

"Perfluorinated aliphatic chain" refers to an aliphatic chain as defined above, wherein every hydrogen atom of the aliphatic chain has been replaced by a fluorine atom. More generally, "perfluorinated" or "perfluoro-", relative to a chemical group, denotes a chemical group, wherein every hydrogen atom of the chemical group has been replaced by a fluorine atom. Examples of $C_1$-$C_{12}$ perfluorinated aliphatic chain include, but are not limited to, trifluoromethyl, pentafluoroethyl (also referred to as "perfluoroethyl"), perfluoropropyl, perfluorobutyl, perfluoropentyl, perfluorohexyl, perfluorocyclopentyl, perfluorocyclohexyl, perfluoroheptyl, perfluorooctyl, perfluorononyl, perfluorodecyl, and perfluorododecyl.

"Solvent" refers to an organic solvent, an inorganic solvent, or a mixture thereof. Examples of organic solvents include, but are not limited to, aliphatic hydrocarbons such as pentane or hexane, alicyclic hydrocarbons such as cyclohexane, aromatic hydrocarbons such as benzene, styrene, toluene, *ortho*-xylene, *meta*-xylene or *para*-xylene, halogenated hydrocarbons such as dichloromethane, chloroform or chlorobenzene, nitrogen-based solvents such as acetonitrile or triethylamine, oxygen-based solvents, in particular ketones such as acetone, ethers such as diethyl ether, methyl tert-butyl ether or tetrahydrofurane (THF) and alcohols such methanol or ethanol, esters or amides, such ethyl acetate or dimethylformamide (DMF), and mixtures thereof. An example of inorganic solvent is water.

"Acid" refers to a Bronsted acid or a Lewis acid, preferably a Bronsted acid. Examples of Bronsted acid include, but are not limited to, hydrochloric acid, hydrobromic acid, hydroiodic acid, sulfuric acid, nitric acid, phosphoric acid, formic acid, acetic acid, trifluoroacetic acid, methanesulfonic acid, benzenesulfonic acid or *p*-toluenesulfonic acid.

## Compounds

The present invention provides a compound represented by the following formula (I):

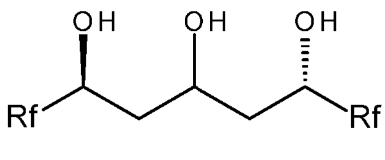

(I),

wherein Rf represents a $C_1$-$C_{12}$ perfluorinated aliphatic chain,
and/or its enantiomer.

In a particular embodiment, Rf represents a $C_1$-$C_6$ perfluorinated aliphatic chain, such as a trifluoromethyl, pentafluoroethyl, perfluoropropyl, perfluorobutyl, perfluoropentyl, perfluorohexyl, perfluorocyclopentyl, or perfluorocyclohexyl.

In another particular embodiment, Rf represents a perfluorinated $C_1$-$C_{12}$ (preferably $C_1$-$C_6$) alkyl, perfluorinated $C_2$-$C_{12}$ (preferably $C_2$-$C_6$) alkenyl, perfluorinated $C_2$-$C_{12}$ (preferably $C_2$-$C_6$) alkynyl, or $C_3$-$C_{12}$ (preferably $C_3$-$C_6$) cycloalkyl, and more particularly, a $C_1$-$C_{12}$ (preferably $C_1$-$C_6$) perfluorinated alkyl.

Preferably, Rf is a trifluoromethyl, a perfluoropropyl or a perfluorobutyl. More preferably, Rf is a trifluoromethyl.

Each of Rf's in a compound of formula (I) may be identical or different. Preferably, all Rf's are identical to each other.

It is understood that the invention pertains to a compound represented by formula (I) and/or its enantiomer, in other words, both enantiomers may be considered separately or in a mixture (for instance, as a racemate). Preferably, both enantiomers are considered separately (i.e. a compound of formula (I) or its enantiomer).

Also, it is understood that "one enantiomer" may refer to a compound existing as one specific enantiomer, or to a mixture of this compound with small amounts of its enantiomer, such that the enantiomeric excess in said mixture is greater than or equal to 96 %, preferably greater than or equal to 98 %, more preferably greater than or equal to 99 %.

The enantiomeric excess "ee" is defined by the following equation (1):

$$ee = | X_+ - X_- | \times 100\ \%, \qquad (1)$$

wherein X+ and X- are mole fractions of enantiomer (+) and enantiomer (-) respectively, with (X+ + X-) = 1.

The enantiomeric purity p+ of enantiomer (+) is defined by the following equation (2):

$$p_+ = X_+ \times 100\ \%, \qquad (2)$$

wherein X+ is as defined above.

The enantiomeric purity p- of enantiomer (-) is defined by the following equation (3):

$$p_- = X_- \times 100\ \%, \qquad (3)$$

wherein X- is as defined above.

The diastereomeric ratio is the ratio of the mole fraction of one diastereomer of a molecule in a mixture to the mole fraction of the other diastereomer(s) of said molecule.

## Process

An object of the invention is a process for preparing a compound of formula (I), comprising the steps of:

(a) reacting 1,3-acetonedicarboxylic acid with an aldehyde of formula Rf-CHO, wherein Rf represents a $C_1$-$C_{12}$ perfluorinated aliphatic chain, or a hydrate thereof, in the presence of a copper salt and a chiral bis(oxazoline) ligand; and

(b) reacting the compound obtained in step (a) with a hydride-type reducing agent.

The conditions (such as temperature, concentration, solvents or equivalents of the reactants) described below for each step may be adjusted by the skilled artisan using his/her general background. Each intermediate or product obtained at the end of a step may be isolated and optionally purified. Alternatively, several steps may be carried out one-pot without isolating said intermediate or product.

### Step (a)

Step (a) of the process according to the present invention comprises reacting 1,3-acetonedicarboxylic acid with an aldehyde of formula Rf-CHO, wherein Rf represents a $C_1$-$C_{12}$ perfluorinated aliphatic chain, in the presence of a copper salt and a chiral bis(oxazoline) ligand. Said aldehyde Rf-CHO used in step (a) may be in the form of a hydrate, i.e. in a form of a compound of formula $Rf\text{-}CH(OH)_2$. Said aldehyde Rf-CHO may be a mixture of one or more aldehydes Rf-CHO (or a hydrate thereof), having different Rf's. In a preferred embodiment, only one aldehyde Rf-CHO (or a hydrate thereof) is used in step (a).

In a particular embodiment, Rf represents a $C_1$-$C_6$ perfluorinated aliphatic chain, such as a trifluoromethyl, pentafluoroethyl, perfluoropropyl, perfluorobutyl, perfluoropentyl, perfluorohexyl, perfluorocyclopentyl, or perfluorocyclohexyl. In another particular embodiment, Rf represents a $C_1$-$C_{12}$ (preferably $C_1$-$C_6$) perfluorinated alkyl, alkenyl, alkynyl, or cycloalkyl, and more particularly, a $C_1$-$C_{12}$ (preferably $C_1$-$C_6$) perfluorinated alkyl. Preferably, Rf is a trifluoromethyl, a perfluoropropyl or a perfluorobutyl. More preferably, Rf is a trifluoromethyl.

The amount of aldehyde Rf-CHO in step (a) may be comprised between 2 and 6 equivalents, preferably between 3 and 5 equivalents, relative to the amount of 1,3-acetonedicarboxylic acid.

According to the present invention, a copper salt refers to an electroneutral compound comprising cationic copper and one or more mono- or poly-atomic, mono- or poly-dentate, organic or inorganic, anionic ligands. The copper salt can be represented by the formula $Cu_xL_y$ wherein:

- L represents a mono- or poly-atomic, mono- or poly-dentate, organic or inorganic, anionic ligand,
- x is 1 or 2, preferably 1, and
- y is 1, 2, or 3, preferably 1 or 2, more preferably 2.

Examples of mono- or poly-atomic, mono- or poly-dentate, organic or inorganic, anionic ligands include, but are not limited to, sulfonates, such as trifluoromethanesulfonate ($O$-$S(O)_2$-$CF_3$), methanesulfonate, or toluenesulfonate, halides such as chloride, bromide, or iodide, carboxylates, such as acetate, or trifluoroacetate, ketyl-ketonates such as acetylacetonate, tetramethylheptanedionate, or hexafluoroacetylacetonate, tetrafluoroborate, pentafluorophosphate, hexafluoroantimonate, nitrite, nitrate, sulfite, sulfate, chlorate, perchlorate, iodate, periodate, hydroxide, superoxide, and carbonate.

In a preferred embodiment, the copper salt is $Cu[O$-$S(O)_2$-$CF_3]_2$.

The amount of copper salt in step (a) may be comprised between 0.01 and 1 equivalent, preferably between 0.05 and 0.5 equivalent, more preferably between 0.05 and 0.2 equivalent, relative to the amount of 1,3-acetonedicarboxylic acid.

The chiral bis(oxazoline) ligand refers to a compound constituted of two chiral oxazoline moieties bound to each other through a linker, such that the bis(oxazoline) ligand is chiral. The chiral bis(oxazoline) ligand can be represented by the following formula (III):

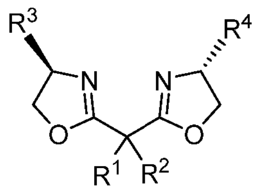
(III),

wherein $R^1$, $R^2$, $R^3$, and $R^4$, independently represent a $C_1$-$C_6$ alkyl group, a $C_2$-$C_6$ alkenyl group, a $C_2$-$C_6$ alkynyl group, a $C_3$-$C_6$ cycloalkyl group, an heterocycloalkyl group, an aryl group, or an heteroaryl group, and/or its enantiomer. Said groups may optionally be substituted by one or more substituents selected from the group consisting of halogen (e.g. chlorine, fluorine, bromine, or iodine), $CF_3$, $C_1$-$C_6$ alkyl, $C_2$-$C_6$ alkenyl, $C_2$-$C_6$ alkynyl, $C_3$-$C_6$ cycloalkyl, -O-($C_1$-$C_6$ alkyl), - NH($C_1$-$C_6$ alkyl) and -N($C_1$-$C_6$ alkyl)($C_1$-$C_6$ alkyl).

In a particular embodiment, $R^1$ and $R^2$ independently represent a $C_1$-$C_6$ alkyl group. Preferably, $R^1$ and $R^2$ are methyl.

In a particular embodiment, $R^3$ and $R^4$ independently represent a $C_1$-$C_6$ alkyl group or an aryl group, preferably an aryl group. More preferably, $R^3$ and $R^4$ are phenyl.

A preferred chiral bis(oxazoline) ligand can be a compound represented by the following formula (III-1):

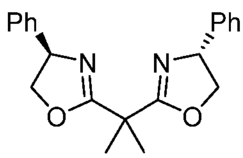
(III-1),

and/or its enantiomer.

Preferably, said chiral bis(oxazoline) ligand is used in the form of one single enantiomer.

The amount of chiral bis(oxazoline) ligand in step (a) may be comprised between 0.02 and 1 equivalent, preferably between 0.05 and 0.5 equivalent, more preferably between 0.1 and 0.2 equivalent, relative to the amount of 1,3-acetonedicarboxylic acid.

In a preferred embodiment, the copper salt and the chiral bis(oxazoline) ligand are added successively or simultaneously in a reaction vessel, and stirred between 15 minutes and 1 hour, before the simultaneous addition of said aldehyde and 1,3-acetonedicarboxylic acid.

Reacting in step (a) may be carried out in any organic solvent, such as tetrahydrofuran. Reacting in step (a) may be carried out at a temperature comprised between 0°C and 50 °C, preferably between 13 °C and 32 °C.

Step (a) produces a compound of formula (II):

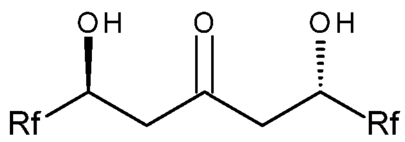

(II),

wherein Rf is as defined in formula (I), and/or its enantiomer.

When the chiral bis(oxazoline) ligand exists as one enantiomer, the enantiomeric excess of the reaction of step (a) is advantageously greater than or equal to 90 %, preferably greater than or equal to 95 %, more preferably greater than or equal to 98 %, as measured by chiral Gas Chromatography (GC). A purification (or resolution in case of a racemate), such as crystallization, may be carried out in order to separate and isolate one compound from its enantiomer.

The enantiomer of a compound of formula (II) obtained by using one given chiral bis(oxazoline) ligand may be prepared by using said copper salt and the corresponding enantiomer of said given chiral bis(oxazoline) ligand. A racemic mixture comprising a compound of formula (II) and its enantiomer may be obtained by using said copper salt with a racemic mixture of said chiral bis(oxazoline) ligand or preferably without said chiral bis(oxazoline) ligand.

Step (b)

Step (b) of the process according to the present invention comprises reacting the compound obtained in step (a), namely a compound of formula (II), with a hydride-type reducing agent.

According to the present invention, a hydride-type reducing agent is a compound having one or more hydrides (formally: " $H^-$ ") that can be transferred to another compound, resulting in the reduction of said other compound (more particularly, a reduction of a ketone group of said other compound into an alcohol group). Examples of hydride-type reducing agent include, but are not limited to, lithium aluminum hydride, $LiAlH(OMe)_3$, sodium or potassium borohydride, zinc borohydride, dihydrogen, borane ($BH_3$), diborane ($B_2H_6$), 9-borabicyclo[3.3.1]nonane (9-BBN), monoisopinocampheylborane, dicyclohexylborane, dimesitylborane, disiamylborane, catecholborane, pinacolborane, L-selectride, and any combination thereof.

In a preferred embodiment, said hydride-type reducing agent is sodium borohydride.

The amount of the hydride-type reducing agent may be comprised between 1 and 4 equivalents, preferably between 1.1 and 2 equivalents, relative to the amount of the compound obtained in step (a).

Reacting in step (b) may be carried out in any organic solvent, such as a mixture of tetrahydrofuran/methanol. It may be carried out, as the whole reaction, at a temperature comprised between -15 °C and 40 °C, preferably between -5 °C and 10 °C.

Step (b) produces a compound of formula (I) and/or its enantiomer. A purification (recrystallization, chromatography...) may be further carried out in order to eliminate all or part of one or more diastereomers of said compound of formula (I) and/or of its enantiomer.

The diastereomeric ratio of the compound obtained at step (b) may be greater than or equal to 95:5, preferably greater than or equal to 98:2, more preferably greater than or equal to 99:1, as measured by $^{19}F$ NMR spectroscopy, or any other technique known by the skilled artisan.

Use as anion-binding agent

A compound represented by formula (I) and/or its enantiomer, is particularly well-suited to be used as anion-binding agent.

More specifically, it is an object of the present invention to use a compound of formula (I) for the enantiomeric separation of chiral anions.

Said chiral anions are typically organic chiral anions.

In a preferred embodiment, said chiral anions are selected from the group consisting of sulfonates, phosphonates, phosphates, hydrogenophosphates, alcoholates, phenolates, thiolates, carboxylates, and combinations thereof,

more preferably phosphates.

An example of phosphate is BINOL-$PO_4$ and can be represented by the following formula:

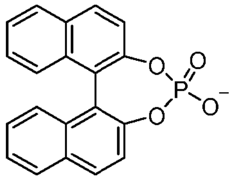

.

The present invention also relates to a separation process, for the enantiomeric separation of chiral anions, comprising the steps of:

(a) contacting a compound of formula (I) with a mixture comprising a pair of enantiomers of a chiral anion, so as to obtain a complex (C) consisting of said compound of formula (I) and one enantiomer of said pair;
(b) isolating said complex (C) obtained in step (a); ; and
(c) optionally recovering the enantiomer from the complex (C) obtained in step (b) .

A "pair of enantiomers of a chiral anion" refers to a mixture of two enantiomers of a chiral anion, said chiral anion having at least one stereogenic atom center (carbon, silicon sulfur stereogenic center) or stereogenic element (chiral axis, helix).

The ratio of said two enantiomers may typically be comprised between 1:99 and 99:1, for instance 50:50 (i.e. racemic mixture).

In said separation process, the compound of formula (I) is advantageously used in the form of one enantiomer.

Step (a) of the separation process produces a complex (C) consisting of said compound of formula (I) and one enantiomer of the pair of enantiomers. The complex is typically formed by coordination of one enantiomer of said pair by said compound of formula (I).

Preferably, the compound of formula (I) used in step (a) selectively forms a complex with one enantiomer of said pair with respect to the other enantiomer of said pair.

In a particular embodiment, a further complex (C') consisting of said compound of formula (I) and the other enantiomer of said pair is obtained in step (a), complexes (C) and (C') being thus diastereomers.

Step (a) may be carried out in any organic solvent such as acetonitrile. Step (a) may be carried out at a temperature comprised between 0 °C and 50 °C, preferably between 15 °C and 35 °C.

In a particular embodiment, the complex (C) in step (a) precipitates.

Step (b) of the separation process comprises isolating the complex (C) obtained in step (a).

In particular, step (b) may comprise separating the complex (C) obtained in step (a) from the other enantiomer of said pair and/or from the further complex (C').

Isolating the complex (C) in step (b) may be carried out by crystallization, chromatography, filtration or centrifugation. In a particular embodiment, complex (C) is isolated by filtration of the precipitate formed in step (a).

In step (c), the enantiomer of the complex (C) obtained in step (b) may be recovered by contacting said complex with any agent that allows the release of said enantiomer from the complex. For instance, said complex may be recovered by ion exchange or by contacting with an acid.

The invention will also be described in further detail in the following examples, which are not intended to limit the scope of this invention, as defined by the attached claims.

EXAMPLES

**General Information**

NMR spectra were recorded on a Bruker AC 300 (300 MHz), a Bruker AC 400 (400 MHz) or a Bruker 500 (500 MHz) spectrometer. Chemical shifts are given in ppm, using as internal standards the residual $CHCl_3$ signal for H NMR ($\delta$ = 7.26) and the deuterated solvent signal for $^{13}C$ NMR ($\delta$ = 77.0). Data for $^{13}C$ NMR are reported as follows: chemical shift (multiplicity). Data for $^1H$ NMR are reported as follows: chemical shift (multiplicity [s = singulet, d = doublet, t = triplet, q = quadruplet, m = multiplet, br = broad], coupling constants *J* in Hertz (Hz), integration). Anhydrous THF was obtained from a Solvent Purification System M Braun SPS-800. High resolution mass spectra (HRMS) were performed on a QStar Elite (Applied Biosystems SCIEX) spectrometer equipped with atmospheric pression ionization source (API) pneumaticly assisted. Samples were ionized by positive electrospray mode as follows: electrospray tension (ISV): 5500 V; opening tension (OR): 50 V; nebulization gas pression (air): 137,9 kPa (20 psi). IR spectra were recorded using a

Thermo Nicolet Avatar 330 FT-IR instrument (Thermo Nicolet Corporation, Madison, WI, USA). Chiral GC analysis were performed on a HP 4890 using 6 bar argon as vector. Column: 25m/0,25 mm. Chromatogram analyzed with ChromNav software. HPLC analyses for the determination of enantiomeric excesses were performed on a Merck-Hitachi system equipped with Chiralpak ASH. Optical rotations were measured at 20 °C in $CHCl_3$ with an Anton Paar MCP 200 polarimeter with a 0.2 cm length. Absolute and relative configuration were determined on compound **3** by X-Ray analysis assuming the same transition states for the other substrates. Chiral anion TBA((*S*)- BINOL-$PO_4$) and TBA((*R*)-BINOL-$PO_4$) were prepared according to a slightly modified literature protocol (Lim, et al, J. Am. Chem. Soc. 2017, 139, 12228 ; Zhou, et al, Arkivoc 2014, 5, 351) using commercially available (*S*)- and (*R*)-BINOL-$PO_4$ and tetrabutylammonium (TBA) hydroxide.

**Example 1**. Preparation of compounds of formula (I)

**General Procedure A — Preparation of compounds of formula (II)**

3.61 mg of copper triflate (0.01 mmol, 10 mol%) and 4.34 mg of (+)-2,2'-Isopropylidenebis[(4*R*)-4-phenyl-2-oxazoline] (0.013 mmol, 13 mol%) were dissolved in 0.1 mL of THF in vial and stirred 30 minutes at room temperature. The corresponding perfluoaldehyde (0.5 mmol, 5 eq) and 16.1 mg of 1,3-acetonedicarboxylic acid were added and the reaction was stirred at room temperature for 4 hours more before addition of 1 mL of 1M HCl and 1 mL of diethyl ether. The aqueous layer the aqueous layer was extracted by 4 times 2 ml of diethyl ether, the combined organic layers filtered over a small plug of $Na_2SO_4$ and silica gel and the solvent evaporated. Purification by recrystallization from a mixture of diethyl ether and *n*-hexane provided the corresponding ketodiol of formula (II).

Compound (1a)

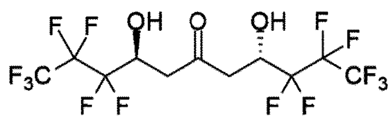

(1a)

Compound (1a) was prepared according to the general procedure A using 99.0 mg of 2,2,3,3,4,4,4-Heptafluorobutyraldehyde hydrate (0.5 mmol, 5 eq), 3.61 mg of copper triflate (0.01 mmol, 10 mol%), 4.34 mg of (+)-2,2'-Isopropylidenebis[(4*R*)-4-phenyl-2-oxazoline] (0.013 mmol, 13 mol%) and 16.1 mg of 1,3-acetonedicarboxylic acid (technical grade, 0.1 mmol, 1 eq). Purification by recrystallization from a mixture of diethyl ether and *n*-hexane provided the corresponding ketodiol (1a). 35.4 mg (0.08 mmol). 78% yield.

The product was obtained in 90% *ee* and 6/1 *dr.*

$[\alpha]^{20}_D$ = +8.72° ($CH_3CN$, c = 0.4), 4/1 *dr*, 90% *ee*).

$^1H$ NMR (400 MHz, $CD_3OD$): $\delta$ (ppm) = 2.83-3.05 (m, 2 *CH2),* 4.71-4.82 (m, 2 C*H*(OH)). $^{13}C$ NMR (100 MHz, $CD_3OD$): $\delta$ (ppm) = 44.1 (2 $CH_2$), 66.3 (dd, *J*= 28.1, 22.7 Hz, 2 *CH(OH)),* 108.7-123.1 (m, 4 *C*($F_2$)), 203.9 (*CO*).

$^{19}F$ (NMR (376 MHz, $CD_3OD$): -82.57 (dd, *J* = 11.5, 8.8 Hz, 2 C$F_3$), -119.97- -121.14 (m, *AB* syst, *J* = 280 Hz, 11.4 Hz, 2 *F*), -125.58- -126.67 (ddd, *AB* syst, *J* = 290.1, 9.1, 4.1 Hz, 2 *F),*-127.40- -128.48 (ddd, *AB* syst, *J*= 290.1, 10.4, 2.6 Hz, 2 *F),* -127.96 -129.11 (m, AB syst, *J* = 280 Hz, 2 *F).*

IR: 688.2, 700.4, 1066.7, 1101.1, 1122.9, 1170.2, 1266.9, 3265.8 $cm^{-1}$

HRMS ESI [M+Na]+ calcd for $C_{11}H_8F_{14}O_3Na$: 477.0142. Observed: 454.0250.

GC enantiomeric excess determination: cyclosil-b; 130°C for 60 min than 1°C/min until 220°C. Retention time (min) = 29.4 min; Retention time (maj) = 31.0 min.

Compound (2a)

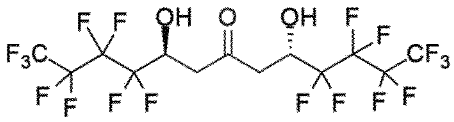

(2a)

Compound (2a) was prepared according to the general procedure A using 99.0 mg of 2,2,3,3,4,4,5,5,5-Nonafluoropentane-1,1-diol (0.5 mmol, 5 eq), 3.61 mg of copper triflate (0.01 mmol, 10 mol%), 4.34 mg of (+)-2,2'-Isopropylidenebis[(4*R*)-4-phenyl-2-oxazoline] (0.013 mmol, 13 mol%) and 16.1 mg of 1,3-acetonedicarboxylic acid (technical grade, 0.1 mmol, 1 eq). Purification by recrystallization from a mixture of diethyl ether and *n*-hexane provided the corresponding ketodiol (2a). 37.7 mg (0.07 mmol). 68% yield.

The product was obtained in 96% *ee* and 30/1 *dr.*

$[\alpha]^{20}_D$ = +6.83° ($CH_3CN$, c = 0.4), 30/1 *dr*, 96% *ee*).

$^1H$ NMR (400 MHz, $CD_3OD$): δ (ppm) = 2.84-3.06 (m, 2 $CH_2$), 4.73-4.87 (m, 2 C*H*(OH)). $^{13}C$ NMR (100 MHz, $CD_3OD$): δ (ppm) = 44.2 (2 *CH2),* 66.7 (dd, *J*= 28.1, 22.7 Hz, 2 *CH(OH)),* 109.4-125.7 (m, 6 *C*($F_2$)), 203.9 (*CO*).

$^{19}F$ (NMR (376 MHz, $CD_3OD$): -82.56 (tt, *J* = 9.8, 3.0 Hz, 2 $CF_3$), -119.49- -120.74 (m, *AB* syst, *J* = 288 Hz, 2 *F*), -122.09- -123.24 (m, *AB* syst, *J* = 298 Hz, 2 *F*), -123.59- -124.82 (m, *AB* syst, *J* = 298 Hz, 2 *F),* -125.97- -127.15 (m, *AB* syst, J = 292 Hz, 2 *F),* -127.15 — -128.21 (m, AB syst, J = 292 Hz, 2 *F*), -127.70- -128.86 (m, *AB* syst, J = 292 Hz, 2 *F).*

IR: 732.9, 1096.9, 1128.8, 1174.6, 1216.4, 1719.9, 3435.5, 3482.2 $cm^{-1}$

HRMS ESI [M+Na]+ calcd for $C_{13}H_8F_{18}O_3Na$: 577.0078. Observed: 577.0079.

GC enantiomeric excess determination: hydrodex-beta-3P; 140°C for 60 min than 1°C/min until 220°C. Retention time (min) = 27.1 min; Retention time (maj) = 28.9 min.

<u>Compound (3a)</u>

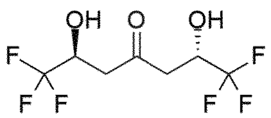

(3a)

Compound (3a) was prepared according to the general procedure A using 99.0 mg of trifluoroacetaldehyde monohydrate (0.5 mmol, 5 eq), 3.61 mg of copper triflate (0.01 mmol, 10 mol%), 4.34 mg of (+)-2,2'-Isopropylidenebis[(4*R*)-4-phenyl-2-oxazoline] (0.013 mmol, 13 mol%) and 16.1 mg of 1,3-acetonedicarboxylic acid (technical grade, 0.1 mmol, 1 eq). Purification by recrystallization from a mixture of diethyl ether and *n*-hexane provides the corresponding ketodiol (3a). 15.6 mg (0.06 mmol). 71% yield.

The product was obtained in 96% *ee* and 30/1 *dr.*

$[\alpha]^{20}_D$ = +24.7° ($CHCl_3$, c = 0.8), 30/1 *dr*, 96% *ee*).

$^1H$ NMR (400 MHz, $CD_3OD$): δ (ppm) = 2.79-3.00 (m, 2 $CH_2$), 4.51-4.62 (m, 2 C*H*(OH)). $^{13}C$ NMR (100 MHz, $CD_3OD$): δ (ppm) = 44.51 (s, 2 $CH_2$), 66.7 (q, *J* = 31.9 Hz, 2 *C*H*(*OH)), 126.69 (q, *J* = 280.9 Hz, 2 $CF_3$), 203.7 (CO).

$^{19}F$ (NMR (376 MHz, $CD_3OD$): -81.35 (s, 2 $CF_3$).

IR: 1088.4, 1128.5, 1166.9, 1264.5, 1300.0, 1728.1, 3326.1, 3389.3 $cm^{-1}$

HRMS ESI [M+Na]+ calcd for $C_{13}H_{24}F_2O_3Na$: 277.0270. Observed: 277.0272.

GC enantiomeric excess determination: cyclosil-b; 110°C for 60 min than 1°C/min until 220°C. Retention time (min) = 81.7 min; Retention time (maj) = 83.1 min.

**General Procedure B — Preparation of compounds of formula (I)**

0.5 mmol (1.0 eq) of the ketodiol (1a, 2a, 3a) were dissolved in 3 mL of dry THF and 0.1 mL of MeOH, and cooled to 0°C under argon. 74 mg of $NaBH_4$ (2 mmol, 2.0 eq) were then added and the reaction was stirred at 0°C for 35 minutes before addition of 0.12 mL of acetic acid. The reaction mixture was then stirred at room temperature for 30 min before addition of 30 mL of saturated aqueous $NaHCO_3$. The mixture was stirred for further 30 min at room temperature before extraction by 3 times 10 mL of diethyl ether, the combined organic layers were washed by 2 times 10 mL saturated

aqueous $NH_4Cl$, 10 mL of saturated aqueous NaCl, 15 mL 1M HCl, 10 mL of saturated aqueous NaCl, dried over $Na_2SO_4$, filtered and the solvent evaporated. Purification by recrystallization from a mixture of diethyl ether and *n*-hexane yielded the pure triol of formula (I).

Compound (1)

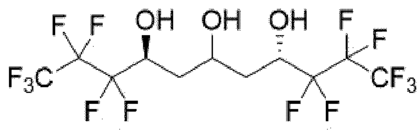

(1)

Compound (1) was prepared according to the general procedure B using 400 mg (0.88 mmol, 1 eq) of the ketodiol (1a) and 65.1 mg of $NaBH_4$ (1.76 mmol, 2 eq). Purification by recrystallization from a mixture of diethyl ether and *n*-Hexane yielded the pure triol (1). 288.0 mg (0.63 mmol). 72% yield. The product was obtained in 97% *ee* and 30/1 *dr.*

$[\alpha]^{20}_D$ = +21.4° ($CH_3CN$, c = 0.4), 30/1 *dr*, 97% *ee*).

$^1H$ NMR (400 MHz, $CD_3OD$): δ (ppm) = 2.75-2.02 (m, 2 C*H₂*), 4.09-4.19 (m, C*H*(OH)), 4.29-4.44 (m, 2 C*H*(OH)). $^{13}C$ NMR (100 MHz, $CD_3OD$): δ (ppm) = 44.1 (2 *CH2),* 66.3 (m, 2 *CH(OH)*), 108.7-123.1 (m, 4 *C*($F_2$)).

$^{19}F$ (NMR (376 MHz, $CD_3OD$): -82.51- -82.58 (m, 2 C*F₃*), -120.68- -121.99 (m, *AB* syst, *J* = 353.4 Hz, 63.8, 26.1 Hz 2F), -125.90- -126.55 (m, *AB* syst, *J* = 290, 47.1, 4.5 Hz, 2F), -127.31--128.38 (m, *AB* syst, *J* = 290.1, 10.7, 1.7 Hz, 2F), -128.21- -129.45 (m, *AB* syst, *J* = 290 Hz, 2*F*).

IR: 733.2, 956.5, 1105.5, 1174.1, 1217.1, 1346.3, 3315.9 $cm^{-1}$
HRMS ESI [M+Na]+ calcd for $C_{11}H_8F_{14}O_3Na$: 479.0299. Observed: 479.0301.

GC enantiomeric excess determination: Lipodex-E; 140°C for 90 min than 1°C/min until 220°C. Retention time (min) = 29.4 min; Retention time (maj) = 31.0 min.

Compound (2)

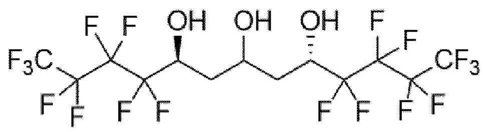

(2)

Compound (2) was prepared according to the general procedure B using 400 mg (0.72 mmol, 1 eq) of the ketodiol (2a) and 53.4 mg of $NaBH_4$ (1.44 mmol, 1.3 eq). Purification by recrystallization from a mixture of diethyl ether and *n*-hexane yielded the pure triol (2). 352.3 mg (0.63 mmol). 88% yield.

The product is obtained in 97% *ee* and 30/1 *dr.*

$[\alpha]^{20}_D$ = +17.8° ($CH_3CN$, c = 1), 30/1 *dr*, 97% *ee*).

$^1H$ NMR (400 MHz, $CD_3OD$): δ (ppm) = 1.70-20.3 (m, 2 C*H₂*), 4.10-4.16 (m, 1 C*H*(OH)), 4.30-4.47 (m, 2 C*H*(OH)). $^{13}C$ NMR (100 MHz, $CD_3OD$): δ (ppm) = 44.0 (2 *CH2),* 66.7 (m, 2 *CH(OH)),* 109.4-125.7 (m, 6 *C*($F_2$)).

$^{19}F$ (NMR (376 MHz, $CD_3OD$): -82.56 (td, *J* = 9.9, 3.1 Hz, 2 C*F₃*), -120.58- -121.63 (m, *AB* syst, *J* = 281.0, 87.5, 22.6, 12.1 Hz, 2 *F*), -122.19 — -123.26 (m, *AB* syst, *J* = 300.0, 57.8, 10.1, 9.5, 4.8 Hz, 2 *F*), -123.60- -124.50 (dq, *AB* syst, *J* = 300.0, 10.3 Hz, 2 *F*), -126.15- -127.09 (m, *AB* syst, *J* = 293.0, 16.3, 7.9, 3.9 Hz, 2 *F*), -127.64- -128.4 (m, *AB* syst, *J* = 281.0 Hz, 2 *F),* - 127.73- -128.66 (m, *AB* syst, *J* = 294.0, 14.6 Hz, 2 *F).*

IR: 720.6, 1086.0, 1130.2, 1198.1, 1216.5, 1354.4, 3344.4 $cm^{-1}$
HRMS ESI [M+Na]+ calcd for $C_{13}H_{24}F_2O_3Na$: 579.0235. Observed: 579.0234.

GC enantiomeric excess determination: hydrodex-beta-3P; 140°C for 60 min than 1°C/min until 220°C. Retention time (min) = 27.1 min; Retention time (maj) = 28.9 min.

Compound (3)

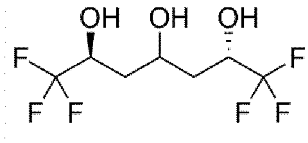

(3)

Compound (3) was prepared according to the general procedure B using 400 mg (1.57 mmol, 1 eq) of the ketodiol (3a) and 116.55 mg of $NaBH_4$ (3.15 mmol, 1.3 eq). Purification by recrystallization from a mixture of diethyl ether and *n*-hexane yielded the pure triol (3). 296.7 mg (1.16 mmol). 74% yield.

The product is obtained in 96% *ee* and 30/1 *dr.*

$[\alpha]^{20}_D$ = +32.4° ($CH_3CN$, c = 0.4), 4/1 *dr,* 90% *ee.*

$^1H$ NMR (400 MHz, $CD_3OD$): δ (ppm) = 1.68-1.89 (m, 2 C*H*$_2$), 4.05-4.23 (m, 3 C*H*(OH)). $^{13}C$ NMR (100 MHz, $CD_3OD$): δ (ppm) = 38.3 (d, *J* = 100.1 Hz, 2 *CH2),* 65.1 (s, *C*H(OH)), 68.1 (m, 2 *C*H*(*OH)), 129.9 (dd, *J*= 281.4, 31.9 Hz, *C*$F_3$), 124.3 (dd, *J* = 281.6, 32.0 Hz, *C*$F_3$) 210.2 (CO).

$^{19}F$ (NMR (376 MHz, $CD_3OD$): -81.48 (d, *J*= 2.6 Hz, 2 C*F*$_3$).

IR: 688.2, 700.4, 1066.7, 1101.1, 1170.2, 1195.5, 1266.9, 3265.8 $cm^{-1}$

HRMS ESI [M+Na]+ calcd for $C_{13}H_{24}F_2O_3Na$: 279.0426. Observed: 279.0428.

GC enantiomeric excess determination: cyclosil-b; 90°C for 0 min than 1°C/min until 220°C. Retention time (min) = 86.1 min; Retention time (maj) = 88.0 min.

**Example 2.** Anion coordination

The anion binding behavior of polyols (1), (2), and (3) was first determined by $^1H$ NMR titration experiments in $CD_3CN$. The addition of tetrabutylammonium chloride (TBACl) or TBA(BINOL-$PO_4$) to polyols resulted in large downfield shifts of the hydroxyl hydrogens. In addition, the binding of fluorinated polyols could also be easily monitored by $^{19}F$ NMR. Association constants were determined using the Thordarson method (P. Thodarson, Chem. Soc. Rev. 2011, 40, 1305 ; D. B. Hibbert, Chem. Commun. 2016, 52, 12792). For each molecule, the titration experiment was repeated three times.

*2.1. Chloride-coordination with compounds (1), (2) and (3)*

A solution of **1** (2.28 mg, 0.005 mmol, 2.5 mM) in $CD_3CN$ (2 mL) was titrated with a solution of TBACl (55.4 mg, 0.2 mmol, 100 mM) in $CD_3CN$ (2 mL) and the $^1H$ NMR signals for the three different hydroxyl hydrogens were monitored (Table 1).

**Table 1**

| **$Cl^-$ equiv.** | **[Cl⁻]** (mol/L) | **[1]** (mol/L) | δ **(OH1)** (ppm) | δ **(OH2)** (ppm) | δ **(OH3)** (ppm) | Δδ **(OH1)** (ppm) | Δδ **(OH2)** (ppm) | Δδ **(OH3)** (ppm) |
|---|---|---|---|---|---|---|---|---|
| **0** | 0 | 0.0025 | 3.45 | 4.05 | 4.34 | 0 | 0 | 0 |
| **0.2** | 0.000508866 | 0.002497502 | 3.82 | 4.41 | 4.57 | 0.37 | 0.36 | 0.23 |
| **0.4** | 0.001026073 | 0.00249501 | 4.2 | 4.79 | 4.81 | 0.38 | 0.38 | 0.24 |
| **0.6** | 0.001539133 | 0.002492522 | 4.49 | 4.99 | 5.1 | 0.29 | 0.2 | 0.29 |
| **0.8** | 0.002091633 | 0.00249004 | 4.76 | 5.16 | 5.38 | 0.27 | 0.17 | 0.28 |
| **1.0** | 0.002590174 | 0.002487562 | 4.97 | 5.29 | 5.59 | 0.21 | 0.13 | 0.21 |
| **1.4** | 0.00353598 | 0.00248139 | 5.26 | 5.48 | 5.89 | 0.29 | 0.19 | 0.3 |
| **2.5** | 0.006148148 | 0.002469136 | 5.45 | 5.6 | 6.07 | 0.19 | 0.12 | 0.18 |
| **3.3** | 0.008137605 | 0.00245942 | 5.46 | 5.62 | 6.09 | 0.01 | 0.02 | 0.02 |

A chloride-coordination constant of 2100 $M^{-1}$ was obtained with compound (1).

A solution of **2** (2.78 mg, 0.005 mmol, 2.5 mM) in $CD_3CN$ (2 mL) was titrated with a solution of TBACl (55.4 mg, 0.2 mmol, 100 mM) in $CD_3CN$ (2 mL) and the $^1H$ NMR signals for the three different hydroxyl hydrogens were monitored (Table 2).

**Table 2**

| $Cl^-$ equiv. | [Cl⁻] (mol/L) | [2] (mol/L) | δ (OH1) (ppm) | δ (OH2) (ppm) | δ (OH3) (ppm) | Δδ (OH1) (ppm) | Δδ (OH2) (ppm) | Δδ (OH3) (ppm) |
|---|---|---|---|---|---|---|---|---|
| **0** | 0 | 0.0025 | 3.45 | 4.05 | 4.33 | 0 | 0 | 0 |
| **0.24** | 0.000608766 | 0.002497502 | 3.83 | 4.43 | 4.56 | 0.38 | 0.38 | 0.23 |
| **0.4** | 0.001009975 | 0.002493766 | 4.11 | 4.71 | 4.74 | 0.28 | 0.28 | 0.18 |
| **0.5** | 0.001320379 | 0.002491281 | 4.43 | 4.94 | 5.04 | 0.32 | 0.23 | 0.3 |
| **0.7** | 0.001815017 | 0.002486325 | 4.7 | 5.11 | 5.31 | 0.27 | 0.17 | 0.27 |
| **1.2** | 0.002882944 | 0.002485089 | 5.1 | 5.38 | 5.73 | 0.4 | 0.27 | 0.42 |
| **1.5** | 0.003581635 | 0.002482622 | 5.19 | 5.43 | 5.81 | 0.09 | 0.05 | 0.08 |
| **2.1** | 0.005275455 | 0.002474023 | 5.29 | 5.49 | 5.9 | 0.1 | 0.06 | 0.09 |
| **3.3** | 0.008137605 | 0.00245942 | 5.46 | 5.62 | 6.08 | 0.17 | 0.13 | 0.18 |
| **4.4** | 0.010821446 | 0.002446184 | 5.5 | 5.64 | 6.12 | 0.04 | 0.02 | 0.04 |

A chloride-coordination constant of 3750 $M^{-1}$ was obtained with compound (2).

A solution of **3** (1.28 mg, 0.005 mmol, 2.5 mM) in $CD_3CN$ (2 mL) was titrated with a solution of TBACl (55.4 mg, 0.2 mmol, 100 mM) in $CD_3CN$ (2 mL) and the $^1H$ NMR signals for the three different hydroxyl hydrogens were monitored (Table 3).

**Table 3**

| $Cl^-$ equiv. | [Cl⁻] (mol/L) | [3] (mol/L) | δ (OH1) (ppm) | δ (OH2) (ppm) | δ (OH3) (ppm) | Δδ (OH1) (ppm) | Δδ (OH2) (ppm) | Δδ (OH3) (ppm) |
|---|---|---|---|---|---|---|---|---|
| **0** | 0 | 0.0025 | 3.45 | 4.05 | 4.34 | 0 | 0 | 0 |
| **0.2** | 0.0005089 | 0.0024975 | 3.82 | 4.41 | 4.57 | 0.37 | 0.36 | 0.23 |
| **0.4** | 0.0010261 | 0.002495 | 4.2 | 4.79 | 4.81 | 0.38 | 0.38 | 0.24 |
| **0.6** | 0.0015391 | 0.0024925 | 4.49 | 4.99 | 5.1 | 0.29 | 0.2 | 0.29 |
| **0.8** | 0.0020916 | 0.00249 | 4.76 | 5.16 | 5.38 | 0.27 | 0.17 | 0.28 |
| **1.0** | 0.0025902 | 0.0024876 | 4.97 | 5.29 | 5.59 | 0.21 | 0.13 | 0.21 |
| **1.4** | 0.003536 | 0.0024814 | 5.26 | 5.48 | 5.89 | 0.29 | 0.19 | 0.3 |
| **2.4** | 0.0061481 | 0.0024691 | 5.45 | 5.6 | 6.07 | 0.19 | 0.12 | 0.18 |

A chloride-coordination constant of 4400 $M^{-1}$ was obtained with compound (3).

*2.2. Chiral anion-coordination with compound (2)*

Experiments of chiral anion-coordination were performed with compound (2) and each enantiomer of BINOL-$PO_4$: (R)-BINOL-$PO_4$ and (S)-BINOL-$PO_4$.

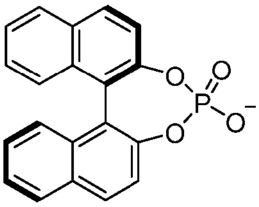

(R)-BINOL-PO4

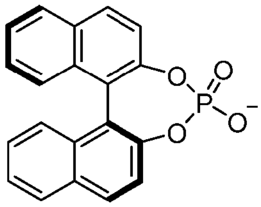

(S)-BINOL-PO4

A solution of **2** (2.78 mg, 0.005 mmol, 2.5 mM) in $CD_3CN$ (2 mL) was titrated with a solution of TBA((*S*)-BINOL-$PO_4$) (30.2 mg, 0.05 mmol, 100 mM) in $CD_3CN$ (0.5 mL) and the $^1H$ NMR signals for the three different hydroxyl hydrogens were monitored (Table 4 and Figure 1).

**Table 4**

| (*S*)-BINOL-$PO_4$ **equiv.** | [(*S*)-BINOL-(mol/L) | **[2]** (mol/L) | δ **(OH1)** (ppm) | δ **(OH2)** (ppm) | δ **(OH3)** (ppm) | Δδ **(OH1)** (ppm) | Δδ **(OH2)** (ppm) | Δδ **(OH3)** (ppm) |
|---|---|---|---|---|---|---|---|---|
| **0** | 0 | 0.0025 | 3.45 | 4.05 | 4.33 | 0 | 0 | 0 |
| **0.4** | 0.000998 | 0.00249501 | 4.61 | 5.01 | 5.23 | 1.16 | 0.96 | 0.9 |
| **0.6** | 0.0014955 | 0.002492522 | 5.01 | 5.25 | 5.64 | 0.4 | 0.24 | 0.41 |
| **0.8** | 0.001992032 | 0.00249004 | 5.32 | 5.43 | 5.97 | 0.19 | 0.18 | 0.33 |
| **1.0** | 0.0024876 | 0.002487562 | 5.56 | 5.56 | 6.2 | 0.24 | 0.13 | 0.23 |
| **1.6** | 0.0037221 | 0.00248139 | 5.77 | 5.9 | 6.55 | 0.21 | 0.34 | 0.35 |
| **2.6** | 0.0061728 | 0.002469136 | 5.87 | 6.04 | 6.72 | 0.1 | 0.14 | 0.17 |

A coordination constant of 8100 (± 35%) $M^{-1}$ was obtained with compound (2) as coordination agent and (S)-BINOL-$PO_4$ as chiral anion.

A solution of **2** (2.78 mg, 0.005 mmol, 2.5 mM) in $CD_3CN$ (2 mL) was titrated with a solution of TBA((*R*)-BINOL-$PO_4$) (30.2 mg, 0.05 mmol, 100 mM) in $CD_3CN$ (0.5 mL) and the $^1H$ NMR signals for the three different hydroxyl hydrogens were monitored (Table 5 and Figure 2).

**Table 5**

| (*R*)-BINOL-$PO_4$**equiv.** | [(*R*)-BINOL-$PO_4$] (mol/L) | **[2]** (mol/L) | δ **(OH1)** (ppm) | δ **(OH2)** (ppm) | δ **(OH3)** (ppm) | Δδ **(OH1)** (ppm) | Δδ **(OH2)** (ppm) | Δδ **(OH3)** (ppm) |
|---|---|---|---|---|---|---|---|---|
| **0** | 0 | 0.0025 | 3.45 | 4.05 | 4.33 | 0 | 0 | 0 |
| **0.2** | 0.0005651 | 0.002497502 | 3.94 | 4.56 | 4.62 | 0.49 | 0.51 | 0.29 |
| **0.4** | 0.0010261 | 0.00249501 | 4.36 | 4.86 | 4.99 | 0.42 | 0.3 | 0.37 |
| **0.6** | 0.0015049 | 0.002492522 | 4.7 | 5.06 | 5.35 | 0.34 | 0.2 | 0.36 |
| **0.8** | 0.001992 | 0.00249004 | 4.98 | 5.22 | 5.64 | 0.28 | 0.16 | 0.29 |
| **1.0** | 0.0024876 | 0.002487562 | 5.25 | 5.38 | 5.93 | 0.27 | 0.16 | 0.29 |
| **1.5** | 0.0037159 | 0.00248139 | 5.61 | 5.66 | 6.34 | 0.36 | 0.28 | 0.41 |
| **2.0** | 0.0052135 | 0.002475248 | 5.72 | 5.83 | 6.53 | 0.11 | 0.17 | 0.19 |
| **2.5** | 0.0064074 | 0.002457002 | 5.81 | 5.97 | 6.68 | 0.09 | 0.14 | 0.15 |
| **3.5** | 0.0094472 | 0.002469136 | 5.92 | 6.1 | 6.85 | 0.11 | 0.13 | 0.17 |

A coordination constant of 1786 (± 12%) $M^{-1}$ was obtained with compound (2) as coordination agent and

(R)-BINOL-$PO_4$ as chiral anion.

The substantial difference between the coordination constants obtained with each enantiomer of BINOL-$PO_4$ demonstrates the ability of the compounds of the invention to selectively coordinate one enantiomer of a chiral anion with respect to the other enantiomer.

## Claims

1. A compound represented by the following formula (I):

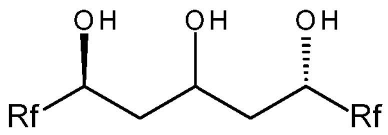

(I),

wherein Rf represents a $C_1$-$C_{12}$ perfluorinated aliphatic chain, and/or its enantiomer.

2. The compound according to claim 1, wherein Rf represents a $C_1$-$C_6$ perfluorinated aliphatic chain, preferably a trifluoromethyl.

3. Use of a compound as defined in claim 1 or 2, for the enantiomeric separation of chiral anions.

4. Use according to claim 3, wherein said chiral anions are selected from the group consisting of sulfonates, phosphonates, phosphates, hydrogenophosphates, alcoholates, phenolates, thiolates, carboxylates, preferably phosphates.

5. A process for the enantiomeric separation of chiral anions, comprising the steps of:

   (a) contacting a compound as defined in claim 1 or 2, with a mixture comprising a pair of enantiomers of a chiral anion, so as to obtain a complex (C) consisting of said compound as defined in claim 1 or 2, and one enantiomer of said pair;
   (b) isolating said complex (C) obtained in step (a); and
   (c) optionally recovering the enantiomer from the complex (C) obtained in step (b).

6. A process for preparing a compound as defined in claim 1 or 2, comprising the steps of:

   (a) reacting 1,3-acetonedicarboxylic acid with an aldehyde of formula Rf-CHO, wherein Rf represents a $C_1$-$C_{12}$ perfluorinated aliphatic chain, or a hydrate thereof, in the presence of a copper salt and a chiral bis(oxazoline) ligand; and
   (b) reacting the compound obtained in step (a) with a hydride-type reducing agent.

7. The process according to claim 6, wherein Rf represents a $C_1$-$C_6$ perfluorinated aliphatic chain, preferably a trifluoromethyl.

8. The process according to claim 6 or 7, wherein said hydride-type reducing agent is sodium borohydride.

9. The process according to any one of claims 6 to 8, wherein said chiral bis(oxazoline) ligand is represented by the following formula (III):

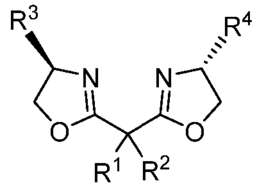

(III),

wherein

$R^1$ and $R^2$ are methyl, and
$R^3$ and $R^4$ are phenyl,

and/or its enantiomer.

**10.** The process according to any one of claims 6 to 9, wherein said copper salt is $Cu[O\text{-}S(O)_2\text{-}CF_3]_2$.

**Patentansprüche**

**1.** Eine Verbindung der folgenden Formel (I):

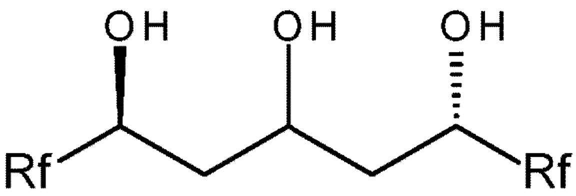 (I),

wobei Rf eine perfluorierte aliphatische $C_1$-$C_{12}$-Kette darstellt und/oder ihr Enantiomer.

**2.** Verbindung nach Anspruch 1, wobei Rf eine perfluorierte aliphatische $C_1$-$C_6$-Kette darstellt, vorzugsweise ein Trifluormethyl.

**3.** Verwendung einer Verbindung nach Anspruch 1 oder 2 zur Enantiomerentrennung chiraler Anionen.

**4.** Verwendung nach Anspruch 3, wobei die chiralen Anionen ausgewählt sind aus der Gruppe bestehend aus Sulfonaten, Phosphonaten, Phosphaten, Hydrogenphosphaten, Alkoholaten, Phenolaten, Thiolaten, Carboxylaten, vorzugsweise Phosphaten.

**5.** Verfahren zur Enantiomerentrennung chiraler Anionen, umfassend die Schritte:

(a) Inkontaktbringen einer Verbindung wie in Anspruch 1 oder 2 definiert mit einer Mischung, umfassend ein Paar von Enantiomeren eines chiralen Anions, um einen Komplex (C) zu erhalten, der aus der Verbindung wie in Anspruch 1 oder 2 definiert besteht, und eines Enantiomers dieses Paares;
(b) Isolieren des in Schritt (a) erhaltenen Komplexes (C); und
(c) gegebenenfalls Gewinnen des Enantiomers aus dem in Schritt (b) erhaltenen Komplex (C).

**6.** Verfahren zur Herstellung einer Verbindung nach Anspruch 1 oder 2, umfassend die Schritte:

(a) Umsetzen von 1,3-Acetondicarbonsäure mit einem Aldehyd der Formel Rf-CHO, wobei Rf eine perfluorierte aliphatische $C_1$-$C_{12}$-Kette darstellt, oder einem Hydrat davon, in Gegenwart eines Kupfersalzes und eines chiralen Bis(oxazolin)-Liganden; und
(b) Umsetzen der in Schritt (a) erhaltenen Verbindung mit einem Reduktionsmittel vom Hydridtyp.

**7.** Verfahren nach Anspruch 6, wobei Rf eine perfluorierte aliphatische $C_1$-$C_6$-Kette darstellt, vorzugsweise ein Trifluormethyl.

**8.** Verfahren nach Anspruch 6 oder 7, wobei das Reduktionsmittel vom Hydridtyp Natriumborhydrid ist.

**9.** Verfahren nach einem der Ansprüche 6 bis 8, wobei der chirale Bis(oxazolin)-Ligand durch die folgende Formel (III) dargestellt wird:

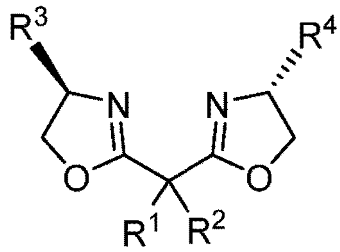

(III),

wobei

$R^1$ und $R^2$ Methyl sind, und
$R^3$ und $R^4$ Phenyl sind

und/oder sein Enantiomer.

**10.** Verfahren nach einem der Ansprüche 6 bis 9, wobei das Kupfersalz $Cu[O-S(O)_2-CF_3]_2$ ist.

**Revendications**

**1.** Composé représenté par la formule (I) suivante :

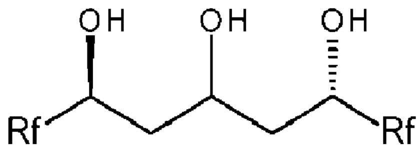

(I),

dans laquelle Rf représente une chaîne aliphatique perfluorée en $C_1$ à $C_{12}$, et/ou son énantiomère.

**2.** Composé selon la revendication 1, dans lequel Rf représente une chaîne aliphatique perfluorée en $C_1$ à $C_6$, de préférence trifluorométhyle.

**3.** Utilisation d'un composé tel que défini dans la revendication 1 ou 2, pour la séparation énantiomérique d'anions chiraux.

**4.** Utilisation selon la revendication 3, dans laquelle lesdits anions chiraux sont choisis dans le groupe constitué par les sulfonates, les phosphonates, les phosphates, les hydrogénophosphates, les alcoolates, les phénolates, les thiolates, les carboxylates, de préférence les phosphates.

**5.** Procédé pour la séparation énantiomérique d'anions chiraux, comprenant les étapes de :

(a) mise en contact d'un composé tel que défini dans la revendication 1 ou 2 avec un mélange comprenant une paire d'énantiomères d'un anion chiral, de façon que soit obtenu un complexe (C) consistant en ledit composé tel que défini dans la revendication 1 ou 2, et un énantiomère de ladite paire ;
(b) isolation dudit complexe (C) obtenu dans l'étape (a) ; et
(c) éventuellement récupération de l'énantiomère à partir du complexe (C) obtenu dans l'étape (b).

**6.** Procédé pour préparer un composé tel que défini dans la revendication 1 ou 2, comprenant les étapes de :

(a) réaction d'acide 1,3—acétonedicarboxylique avec un aldéhyde de formule Rf— CHO dans laquelle Rf représente une chaîne aliphatique perfluorée en $C_1$ à $C_{12}$, ou un hydrate de celui—ci, en présence d'un sel de cuivre et d'un ligand bis(oxazoline) chiral ; et
(b) réaction du composé obtenu dans l'étape (a) avec un agent réducteur de type hydrure.

7. Procédé selon la revendication 6, dans lequel Rf représente une chaîne aliphatique perfluorée en $C_1$ à $C_6$, de préférence trifluorométhyle.

8. Procédé selon la revendication 6 ou 7, dans lequel ledit agent réducteur de type hydrure est le borohydrure de sodium.

9. Procédé selon l'une quelconque des revendications 6 à 8, dans lequel ledit ligand bis(oxazoline) chiral est représenté par la formule (III) suivante :

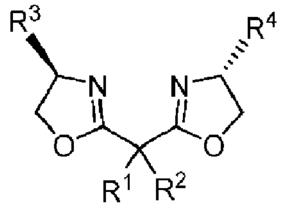 (III),

dans laquelle

$R^1$ et $R^2$ sont méthyle, et
$R^3$ et $R^4$ sont phényle,
et/ou son énantiomère.

10. Procédé selon l'une quelconque des revendications 6 à 9, dans lequel ledit sel de cuivre est $Cu[O—S(O)_2—CF_3]_2$.

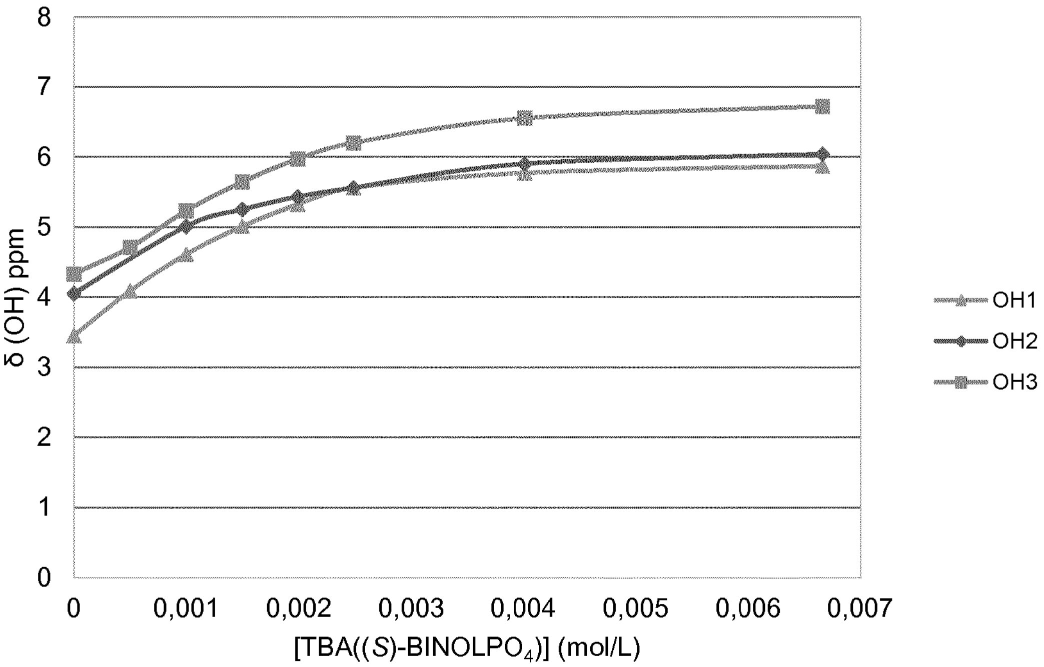


**Figure 1**

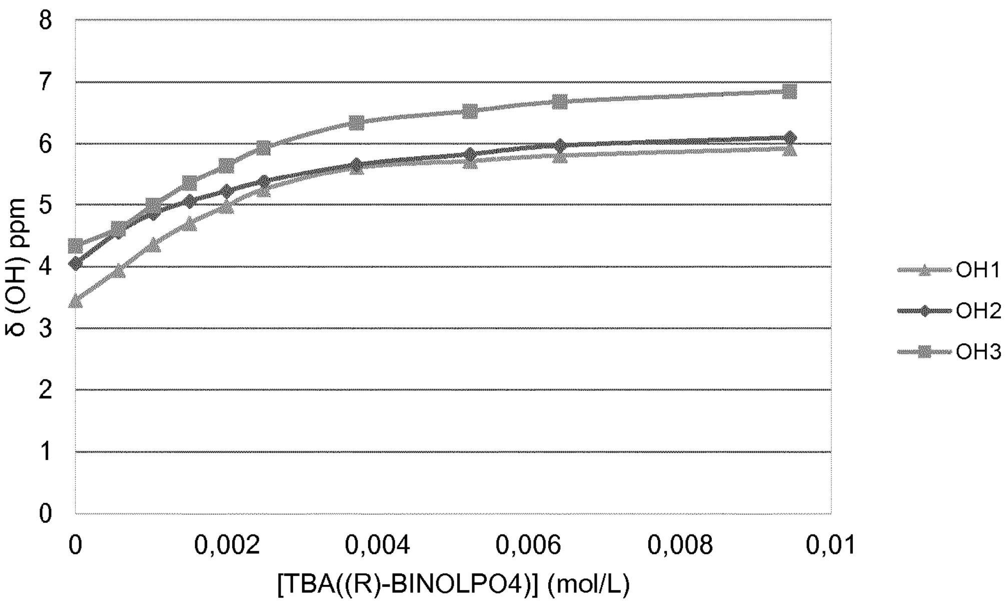


**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PIRKLE et al.** *J. Org. Chem.,* 1977, vol. 42, 384-387
- **SAMET et al.** *J. Org. Chem.,* 2015, vol. 80, 1130-1135
- **SHOKRI et al.** *J. Am. Chem. Soc.,* 2013, vol. 135, 9525-9530
- **LIM et al.** *J. Am. Chem. Soc.,* 2017, vol. 139, 12228
- **ZHOU et al.** *Arkivoc,* 2014, vol. 5, 351
- **P. THODARSON.** *Chem. Soc. Rev.,* 2011, vol. 40, 1305
- **D. B. HIBBERT.** *Chem. Commun.,* 2016, vol. 52, 12792